# EUROPEAN PATENT SPECIFICATION

(11) **EP 3 142 545 B1**
(45) Date of publication and mention of the grant of the patent: **07.08.2019**
(21) Application number: 15729531.2
(22) Date of filing: 05.05.2015
(51) Int. Cl.: A61B 5/00

(54) **AN APPARATUS FOR A WEARABLE DEVICE**
VORRICHTUNG FÜR EINE AM KÖRPER TRAGBARE VORRICHTUNG
APPAREIL DESTINÉS À UN DISPOSITIF PORTABLE

(30) Priority: 15.05.2014 GB 201408635; 14.08.2014 GB 201414396
(43) Date of publication of application: 22.03.2017
(73) Proprietor: Nokia Technologies Oy, 02610 Espoo (FI)
(72) Inventor: SALO, Antti, FI-08500 Lohja as. (FI); ROUVINEN, Jarkko, FI-02340 Espoo (FI); KOSONEN, Matti, FI-04420 Järvenpää (FI)
(74) Representative: Swindell & Pearson Limited
(86) International application number: PCT/FI2015/050300
(87) International publication number: WO 2015/173464

(56) References cited:
- EP-A1- 0 315 040
- EP-A1- 1 691 190
- EP-A1- 2 689 721
- EP-A2- 1 070 479
- DE-B3-102006 005 211
- US-A- 5 243 992
- US-A- 5 263 244
- US-A1- 2007 206 655
- US-A1- 2013 187 789

## Description

### TECHNOLOGCAL FIELD

Examples of the present disclosure relate to an apparatus, method and computer program for a wearable device and method for the same. In particular, though without prejudice to the foregoing, certain examples relate to an apparatus, method and computer program for a wrist device.

### BACKGROUND

Conventional wrist devices comprising electronic hardware including sensors are not always optimal. Difficulties can arise in providing electrical hardware and electronic components in a curved wrist device which is adequately robust as well as comfortable for prolonged wearable use. Typically, electrical hardware and electronic components such as sensors are mounted on rigid circuit boards and are thus limited in their position within the wrist device to rigid planar portions of the wrist device.

The listing or discussion of any prior-published document or any background in this specification should not necessarily be taken as an acknowledgement that the document or background is port of the state of the art or is common general knowledge. One or more aspects/examples of the present disclosure may or may not address one or more of the background issues.

US 2013/0187789 discloses wrist-worn device that monitors movements of a user. A sensor assembly of the wrist-worn device is configured to detect movement of the user and generate sensor data based on the movement detected. A controller connected to the sensor assembly obtains movement data based on the sensor data. An antenna connected to the controller is configured to operate at a desired frequency when a wrist of the user is received by the device such that the movement data is wirelessly transmittable from the wrist-worn device to an electronic device. The antenna may exhibit a different design and configuration depending on the size of the wrist-worn device.

A device according to the preamBle of claim 1 is known from EP 2 689 721 A1.

### BRIEF SUMMARY

The present invention is as set out in the independent claim.

According to at least some but not necessarily all examples of the disclosure there is provided an apparatus comprising:
a support structure configured to at least partially enclose an appendage of a user, said support structure defining an inner side and an outer side; and
a first section of flexible circuitry disposed towards the inner side of the support structure.

The apparatus is configured for wearable use on the user's appendage, e.g. limb. For example, the apparatus may be configured as a wrist device that wraps around and can be worn on a user's wrist.

The first section of flexible circuitry may comprise one or more sensors. The first section of flexible circuitry is separate from the support structure, and is provided in a wearable sensor element which is separate from the support structure.

The support structure comprises a first attachment mechanism for attaching the support structure to the appendage which is different from a second attachment mechanism of the wearable sensor element. For example, the first attachment mechanism of the support structure may be a first bracelet, whereas the second attachment mechanism of the wearable sensor element may be a second bracelet separate and different to the first bracelet. Alternatively, an adhesive based attachment mechanism could be used for the wearable sensor element. Advantageously, such a modular/multi device arrangement enables an attachment mechanism to be provided to each module such that, for example, a thin and light weight wearable sensor element may be adhered to a user's wrist or provided as a tight fitting bracelet, whereas the heavier support structure (housing the electronics/circuitry and power supply for controlling the wearable sensor element) may be provided as a bracelet having a looser fit to improve user comfort.

According to at least some but not necessarily all examples of the disclosure there is provided an apparatus comprising:
a wearable sensor element configured to attach to a user's appendage; and
a wearable structure, separate from the wearable sensor element, configured to at least partially enclose and attach to the appendage;
and wherein the wearable structure comprises electronics for controlling the wearable sensor element.

According to at least some but not necessarily all examples of the disclosure there is provided an apparatus comprising:
support means configured to at least partially enclose an appendage of a user, said support means defining an inner side and an outer side; and
first flexible circuitry means disposed towards the inner side of the support means.

According to at least some but not necessarily all examples of the disclosure there is provided a method for operating the apparatus, comprising:
detecting a manipulation of the apparatus; and
controlling operation of the apparatus in dependence on the detected manipulation.

According to at least some but not necessarily all examples of the disclosure there is provided a computer program that, when performed by at least one processor, causes the above method to be performed.

According to at least some but not necessarily all examples of the disclosure there is provided a non-transitory computer readable medium encoded with instructions that, when performed by at least one processor, causes the above method to be performed.

### BRIEF DESCRIPTION OF THE DRAWINGS

For a better understanding of various examples that are useful for understanding the detailed description reference will now be made by way of example only to the accompanying drawings in which:
Figure 1 schematically illustrates an example of an apparatus according to the present disclosure;
Figure 2 schematically illustrates an example of a circuitry arrangement for an apparatus according to a first aspect of the present disclosure;
Figure 3 schematically illustrates a further example of an apparatus according to the first aspect of the present disclosure;
Figure 4 schematically illustrates a yet further example of an apparatus according to the first aspect of the present disclosure;
Figure 5 schematically illustrates a yet further example of an apparatus according to the first aspect of the present disclosure;
Figure 6 schematically illustrates an example of a controller for an apparatus according to the present disclosure;
Figure 7 illustrates a method according to an example of the present disclosure;
Figure 8 schematically illustrates an example of an apparatus according to a second aspect of the present disclosure;
Figures 9A and 9B schematically illustrate plan and side on views of a further example of an apparatus according to the second aspect of the present disclosure; and
Figures 10A-D schematically illustrate relative positions of a wearable structure and a separate wearable sensor element of further examples of an apparatus according to the second aspect of the present disclosure.

### DETAILED DESCRIPTION

The Figures schematically illustrate an apparatus 100 comprising a support structure/mechanical support 101 configured to at least partially enclose an appendage of a user, e.g. a user's wrist. The support structure defines an inner side 102. The apparatus also comprises a first section of flexible circuitry 103, e.g. flexible wiring board, which is disposed towards the inner side 102 of the support structure.

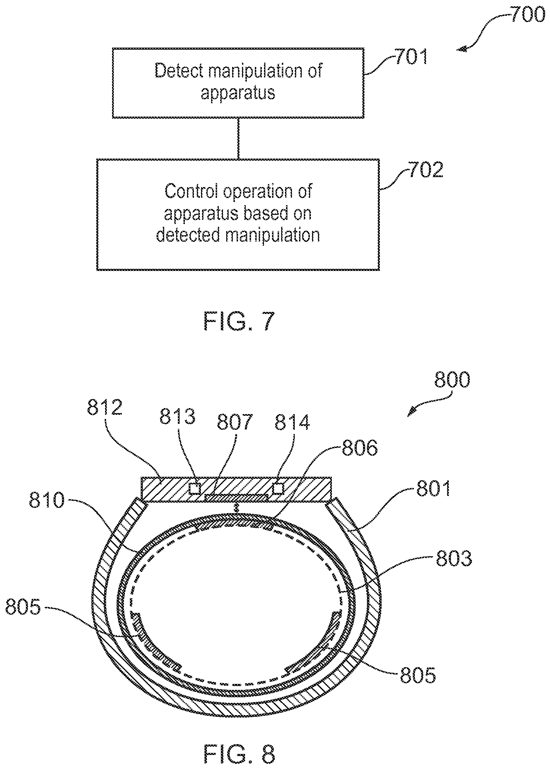

In certain particular examples of the present disclosure, the first section of flexible circuitry 103 comprises circuitry mounted/printed/integrated thereon, such as a first sensor 105 that is inwardly facing 104 on the inner side 102 of the support structure 101. Advantageously, the sensor 105 is not limited in its locations within the apparatus 100 to rigid/planar flat portions of the apparatus. Instead, the sensor 105 itself can be flexible/curved so as to conform to the shape of the support structure and the user's appendage and can be disposed anywhere along the length of the first section of flexible circuitry 103 which itself can be located anywhere along the curved inner side 102 of the support structure 101. The inner sensor 105 can be in direct physical contact with, or immediately proximal to, the user's skin/wrist. Advantageously, such examples provide improved integration of sensors in a curved device and also provide improved sensing/monitoring of the user. As will be discussed in greater detail below with regards to the second aspect of the disclosure, the first section of flexible circuitry 103 may be separate of the support structure, i.e. not a part of the support structure but instead a part of a separate wearable sensor element which is separate and distinct from the support structure (e.g. as shown in Figure 8).

In certain other particular examples of the present disclosure, a second section of flexible circuitry 107 is additionally provided which comprises circuitry mounted/printed/integrated thereon, such as a second sensor 109 that is outwardly facing 108 on the outer side 106 of the support structure 101. The sensor 109 itself can be flexible/curved and can be outwardly disposed anywhere along the length of the curved outer side 106 of the support structure 101. Thus the sensor 109 is not limited to locations within the apparatus 100 that are rigid and planar. Moreover, the outer sensor 109 could be directly exposed to the environment thereby being optimally arranged to monitor the external environment. Such examples again provide improved integration of sensors and electrical hardware in a curved device and also provide improved sensing/monitoring of both a user and the environment.

Certain other examples provide flexible inner 103 and outer 107 sensor circuitries that can be provided with an array of integrated/printed sensors thereon so as to substantially cover the inner 102 and outer sides 106 of the support structure 101 thereby increasing the sensing surface area of the device.

Examples of apparatuses according to the present disclosure will now be described with reference to the Figures. Similar reference numerals are used in the Figures to designate similar features. For clarity, all reference numerals are not necessarily displayed in all figures.

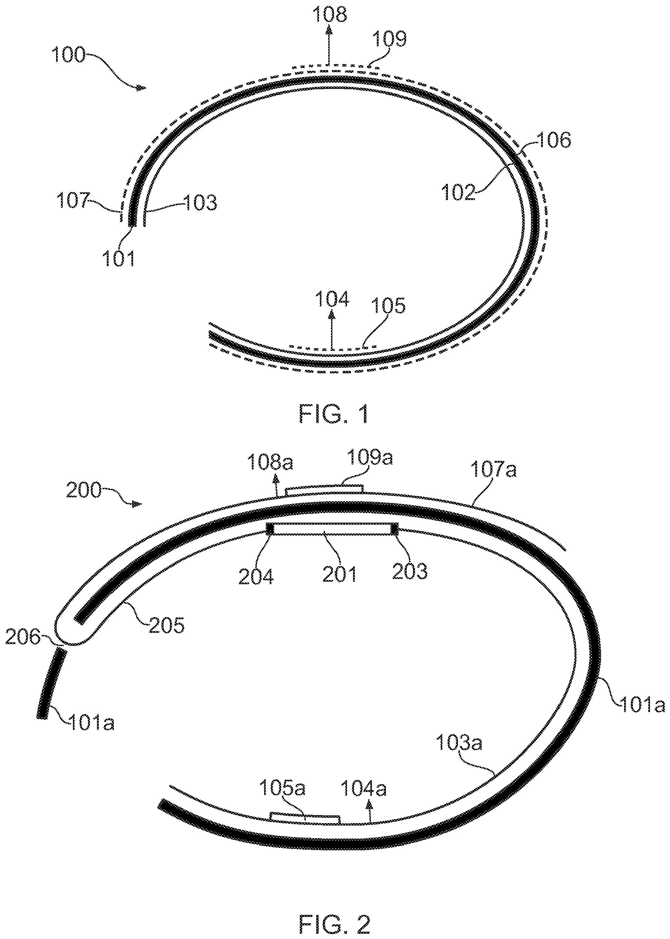

Figure 1 schematically illustrates a cross sectional view of an apparatus 100 according to an example of the present disclosure. Figure 1 focuses on the functional components necessary for describing the operation of the apparatus.

The apparatus 100 comprises: a support structure 101 and a first section of flexible circuitry 103.

The support structure 101 provides an overarching framework for the apparatus and mechanical support for components of the apparatus, e.g. user interface, electrical components, control circuitry and power supply for the apparatus and the first section of flexible circuitry 103. The support structure may be: a mechanical architecture, support means, support member, a frame, a chassis or a skeleton structure which forms the back bone of the apparatus. The support structure 101 is configured to at least partially enclose, envelope or wrap around a user's appendage, e.g. a user's wrist (not shown), such that the apparatus may be worn by the user around the user's appendage, for example in the form of a band, bracelet or strap for wearing on a wrist or other body location. In this regard, the support structure 101 is curved, having a curvilinear cross section substantially of a generally oval/circular/band like shape. In the example shown, the apparatus has a bracelet/band like form-factor albeit not forming a closed loop so as to have a "C" shaped cross section.

The support structure 101 defines an inner side 102 / inward direction 104 and an outer side 106 / outward direction 108. When the device is worn by a user, the inner/interior 102 side corresponds to a side which is proximal to the user's appendage whilst the outer/exterior side corresponds to a side which is distal from the user's appendage.

The first section of flexible circuitry 103 may comprise, for example: a flexible wired board, flexible circuit board or flexible printed circuit board. The first section of flexible circuitry 103 is disposed towards the inner side 102 of the support structure 101. For example the first section could be disposed on the inner side 102. In a first aspect of the disclosure, the first section of flexible circuitry 103 is coupled to and supported by an inner surface of the support structure 101. The first section of flexible circuitry 103 extends around a substantial portion of the inner side 102 of the support structure 101. In the example shown, the first section of flexible circuitry 103 covers the entirety of the inner surface of the support structure 101. In other examples, the first section of flexible circuitry may extend to cover at least a portion or full length of the inner circumference of the inner side 102. As will be discussed in greater detail below, e.g. with regards to Figure 8, in a second aspect of the disclosure, the first section of flexible circuitry may be separate and distinct from the support structure, e.g. provided as a separate device such as a wearable sensor element, and the support structure may comprise electronics, circuitry and a power supply for controlling the wearable sensor element.

The first section of flexible circuitry 103 may comprise circuitry mounted thereon, e.g. surface mount device (SMD) components mounted thereon or circuitry printed or integrated thereon, such as at least a first sensor 105 or an array of sensors. Advantageously, such circuitry/sensors are themselves flexible and can be curved so as to conform to the curved shape of the support structure and the shape of the user's appendage so as to provide a comfortable wearable apparatus. Moreover, advantageously, since the sensor can be printed on the flexible circuitry, the location of the sensor is not limited to flat/planar sections of circuitry and the sensor could be located at any point on the flexible circuitry and any point on the inner side. Furthermore, the size and number of sensors are not limited to that able to be fitted into flat/planar sections. Thus, advantageously, examples of the present disclosure enable larger sensing surface areas and more numerous sensors to be used than typically possible for conventional wrist devices.

The sensors may be configured so as to be able to be in direct physical contact with the user's appendage/skin (or at least immediately proximal to the user's skin, i.e. with merely a thin protective layer covering the sensor). The at least first sensor 105 may be inwardly disposed 104 on an inner/innermost side of the apparatus 100. The at least first sensor 105 is thus optimally arranged to sense conditions on an inner side 102 of the apparatus 100 proximal to the user's appendage and monitoring the user, i.e. measure and detect conditions on an internal side of the apparatus 100.

The at least first sensor 105 or array of sensors may be configured to extend/ be disposed along a substantial portion of the first flexible circuitry section 103. The at least first sensor 105 or array of sensors may comprise a plurality of the same type of sensor or differing types of sensor. Types of first sensors that may be used include, but are not limited to: heart rate, blood pressure, blood glucose, humidity, temperature, galvanic skin response and skin moisture or other sensors to monitor the user. In addition or instead of the sensors, a device for taking blood samples from or controlling medicine injection to the user's appendage may be provided, such a device being controlled by electronics and circuitry housed in the support structure.

A second section of flexible circuitry 107 (shown in outline) may additionally be provided that is disposed towards the outer side 106 of the support structure 101. The second section of flexible circuitry 107 is coupled to and supported by an outer surface of the support structure 101. The second section of flexible circuitry 107 may extend around a substantial portion of the outer side 106 of the support structure 101. In the example shown, the second section of flexible circuitry 107 covers the entirety of the outer surface of the support structure 101. In other examples, the second section of flexible circuitry may extend to cover at least a portion or full length of the outer circumference of the outer side.

The second section of flexible circuitry 107 may comprise circuitry mounted thereon, e.g. SMD components mounted thereon or circuitry printed or integrated thereon, such as at least a second sensor 109 or an array of sensors. The at least second sensor 109 may be outwardly disposed 108 on an outer/outermost side 106 of the apparatus 100 so as to be directly exposed to the external environment (or at least merely covered by a protective layer). The at least second sensor 109 is thus optimally arranged to sense external conditions on an outer side 106 of the apparatus 100 distal from a user's appendage and thus monitor the external environment, i.e. measure and detect conditions external of the apparatus 100 or other sensors to monitor an external environment (cf. monitoring the user).

The at least second sensor 109 or array of sensors may be configured to extend/ be disposed along a substantial portion of the second flexible circuitry section 107. The at least second sensor 109 or array of sensors may comprise a plurality of the same type of sensor or differing types of sensor. Types of second sensors that may be used include, but are not limited to: humidity, temperature, touch, strain, stretch, bend, compression, as well as contortion or user manipulation of the apparatus 100.

Advantageously, certain examples of the present disclosure provide a wrist device comprising at least a first sensor optimally arranged to monitor a user of the apparatus, and at least a second sensor optimally arranged to monitor an external environment.

The apparatus 100 may be provided in a module. As used here 'module' refers to a unit or apparatus that excludes certain parts/components that would be added by an end manufacturer or a user.

Figure 2 illustrates a cross sectional view of an example of a circuitry arrangement 200 for an apparatus according to a first aspect of the present disclosure (in this figure, a support structure is not illustrated).

The circuitry arrangement 200 comprises a first flexible circuitry section 103a, which is disposed towards an inner side of a support structure 101a, and a second flexible circuitry section 107a, which is disposed towards an outer side of the support structure.

Circuitry 105a, such as at least a first sensor, may be printed onto or integrated into the first flexible circuitry section 103a and disposed towards an inwardly facing side of the first flexible circuitry section, e.g. as indicated at 104a. Such a first flexible circuitry section 103a comprising at least a first sensor may be referred to as an inner sensor foil. Likewise, circuitry 109a, such as at least a second sensor, may be printed onto or integrated into the second flexible circuitry section 107a and disposed towards an outwardly facing side of the second flexible circuitry section, e.g. as indicated at 108a. Such a second flexible circuitry section 107a comprising at least a second sensor may be referred to as an outer sensor foil.

Although in Figure 2 a gap is shown between the inner sensor foil and the support structure and a gap is shown between the outer sensor foil and the support structure, it is to be appreciated that in embodiments of the apparatus according to the first aspect of the disclosure the inner sensor foil may be disposed on and in contact with the inner surface of the support structure, e.g. supported there on and adhered thereto. Similarly the outer sensor foil may be disposed on and supported by an outer surface of the support structure. However, according to a second aspect of the disclosure, portions of the inner and outer flexible foils may be separate and distinct from the support structure and provided in a separate wearable sensor element. The support structure may comprise electronics, circuitry and a power supply for controlling the wearable sensor element.

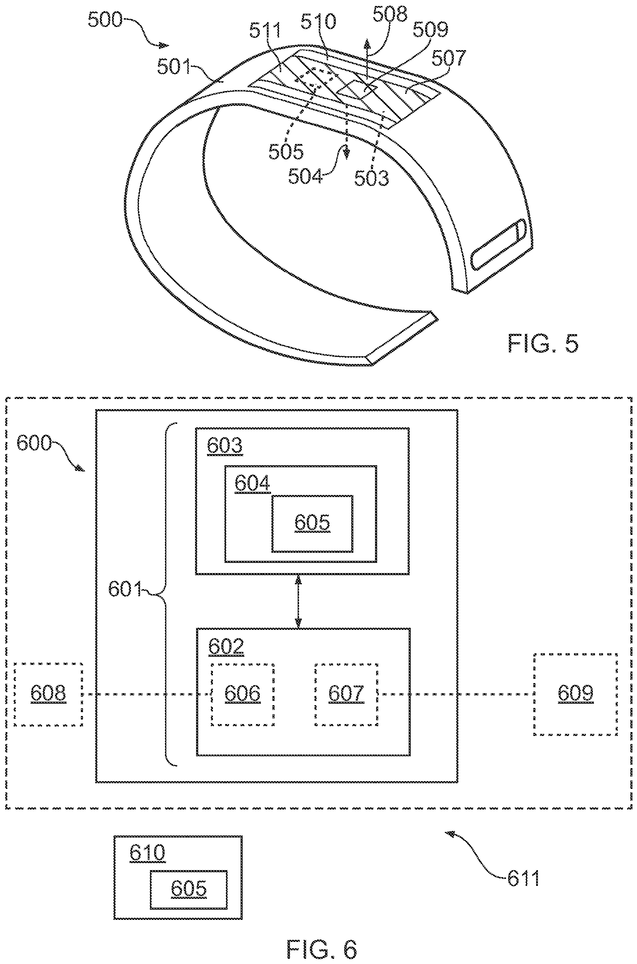

The first flexible circuitry section 103a is electrically connected to circuitry 201 via electrical connector 203. The second flexible circuitry section 107a, which is disposed towards an outer side of the support member, is electrically connected to the circuitry 201 via a portion of second flexible circuitry 205 which passes through an aperture, slot or opening 206 in the support structure, such that the portion of the second flexible circuitry 205 is disposed on the inner side of the support structure) and electrical connector 204, such that the circuitry 201 is interposed between the first and second flexible circuits. The circuitry 201 may be inherently rigid, i.e. a rigid: circuit board, printed circuit board or printed wired board. Alternatively, instead of having a rigid planar flat substrate, the circuitry could be rigidly supported by a rigid planar flat surface of the support structure. Advantageously, providing a rigid flat/planar circuitry section facilitates the mounting and integration of electrical hardware and components, for example for electrical hardware, such as a controller as discussed with reference to Figure 6 or other circuitry, components, devices and sensors (e.g. accelerometers, GPS receivers), not suitable for printing on the flexible circuitry.

The first and second flexible circuitry sections 103a and 107a could correspond to separate / different flexible circuits. The material of the flexible substrate of the first flexible circuitry section 103a may be different to the material of the flexible substrate of the second flexible circuitry section 107a. The substrate materials may include: Polymethyl methacrylate (PMMA), Polyethylene terephthalate (PET) and other low temperature process plastics which would be well suited for accommodating printed electronics.

Alternatively, the first and second flexible circuitry sections 103a and 107a along with an intermediate circuitry section could be integrally formed as a single unit, with the intermediate circuitry section being provided by rigid support from the support structure or by the provision of a rigid substrate. The rigid support may be in the form of a flat planar rigid support. In such an example where the first and second flexible circuitry sections 103a and 107a are integrally formed as a single unit with circuitry 201, the connectors 203 and 204 are not required.

In a yet further example, the first and second flexible circuitry sections 103a and 107a could correspond to first and second sides of the same single flexible circuit, which is appropriately wrapped around the support structure so as to have a first section 103a disposed towards an inner side and a second section 107a disposed towards an outer side.

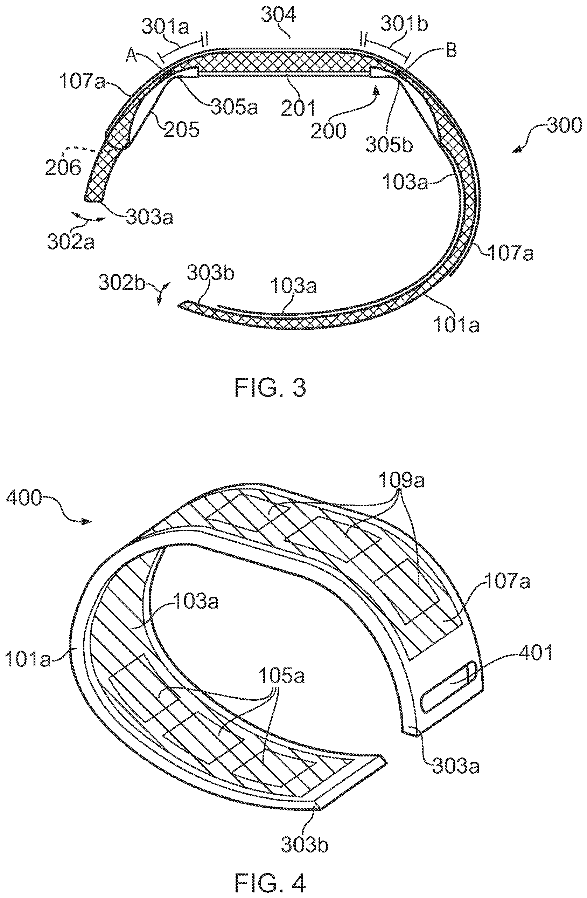

Figure 3 illustrates a cross sectional view of a further example of an apparatus 300 according to the first aspect of the present disclosure. The apparatus 300 comprises a support structure 101a, and the circuitry arrangement 200 of Figure 2 comprising an inner sensor foil, i.e. first flexible circuitry section 103a, an outer sensor foil, i.e. second flexible circuitry section 107a, and an interposing rigid circuitry section 201. Electrical components and electrical hardware, such as a controller, processor and memory, may be mounted and integrated in the rigid circuitry portion.

The inner and outer sensor foils can be coupled to the support structure via any suitable means, for example adhered by double sided adhesive tape. The support structure is configured such that it has portions of varying rigidity. The support structure 101a has flexible portions 301a and 301b which may be bent upon user actuation, e.g. a user squeezing the apparatus between thumb and forefinger, so as to enable movement of the limb/end portions 303a and 303b of the support structure as represented with double headed arrows 302a and 302b. The flexible portions may also allow rotation of the limb/end portions 303a and 303b about points A and B respectively. Such flexibility provides improved user comfort, but can also be used for user input.

One of the sensor foils, e.g. the outer sensor foil, may comprise one or more sensors, such as a strain sensor to detect such user actuation/manipulation of a part of the apparatus such as the support structure, namely the user: squeezing or flexing the apparatus to cause relative movement or rotation of parts of the support structure. A signal from such a sensor, indicative of such user manipulation of the apparatus/support, could be used for a user input or user interface command. For example, the signal from such a sensor could be provided to a controller circuitry mounted on the rigid circuitry 201 and used as an input command for a user interface or to control the apparatus in dependence on detection of user actuation of the bendable section.

The apparatus 300 is provided with means configured to withstand a degree of bending applied by the user to the support structure. In the example of Figure 3, such means correspond to the provision of an extra length, i.e. slack 305a and 305b, in the first and second flexible circuitries to allow for/accommodate bending of sections of the support structure. Means configured to withstand a degree of bending of the apparatus could relate to the selection of materials for parts of the apparatus, e.g. resilient materials, or the structure of the apparatus, e.g. sections of reduced width 301a and 301b to permit a degree of bending.

Means configured to limit a degree of bending able to be applied to the support structure could also be provided. For example, the rigid section of the support structure could be provided with a protruding member configured to mate/engage with a corresponding recession in the flexible section such that bending of the apparatus causes the protrusion to enter into and engage with the recession. A gap is provided between the recession and the protrusion to permit a degree of movement/bending of the apparatus, but limit the degree of bending i.e. when the protrusion fully engages with and abuts against recession thereby preventing any further movement and bending.

The two flexible/user manipulable sections are located on either side of a rigid section 304. The rigid section 304 provides a rigid and non-flexible support area for the rigid circuitry 201. This provides a secure and robust housing for electrical components and hardware mounted therein to protect delicate circuitry and main electronic components, such as control circuitry of the apparatus.

The rigid, bendable and flexible portions could be achieved by any suitable means, e.g. providing relatively thicker and thinner portions of the support structure or making such portions of the support structure from materials having the appropriate mechanical characteristics. Alternatively a mechanism could be provided to enable bending, such as a hinge.

Figure 4 illustrates a yet further example of an apparatus 400 according to the first aspect of the present disclosure. The apparatus broadly corresponds to the apparatus 300 of Figure 3, i.e. comprising a support structure 101 a, inner sensor flex 103a on an inner side of the support structure and an outer sensor flex 107a on an outer side of the support structure. Figure 4 also shows a plurality of printed sensors 105a on the inner surface of the inner sensor flex and a plurality of printed sensors 109a on the outer surface of the outer sensor flex. The inner and outer sensor flexes may be provided with sensors substantially covering their respective surfaces.

The printed sensors and the inner and outer sensor flexes may be provided with a protective layer, i.e. a protective layer overlying the inner surface of the inner sensor flex and protective layer overlying the outer surface of the outer sensor flex.

A fastening device may be used to secure and tighten the apparatus around the user's limb. In the example of Figure 4, an aperture 401 is provided in one of the limb end portions 303a of the apparatus. A strap (not shown), which is secured to the other limb end portion 303b, can be passed through the aperture 401 and used to fasten and tighten the apparatus around the user's appendage. For example a hook and loop based fastening strap may be used in this regard. Alternatively, other fastening and/or clasping means may be used.

The second flexible circuitry section 107a, which is disposed on an outer side of the support structure 101a, may be electrically connected to the other circuitry (e.g. circuitry 201 not shown in Figure 4) which is disposed on the inner side of the support structure. Such electrical connection is provided via a portion of second flexible circuitry (e.g. portion 205 not shown in figure 4) which is disposed on an inner side of the support structure. The support structure may comprise an aperture (e.g. aperture 206 not shown in Figure 4) through which the second flexible circuitry can pass to enable the second flexible circuitry to wrap around the support structure and thereby have a section of the second flexible circuitry which is disposed on the outer side of the support structure as well as a portion disposed on the inner side of the support structure for electrical connection to other circuitry.

Advantageously, placing the other circuitry 201 on an inner side of the support structure helps protect the circuitry and the main electrical components of the apparatus from the external environment. However, in an alternative example, the circuitry 201 may be provided on the outer side of the support structure, such that an equivalent aperture and wrap around arrangement would be provided for the first flexible circuitry to enable the first flexible circuitry section 103a, disposed on an inner side of the support structure 101a, to be electrically connected to circuitry 201 disposed on the outer side of the support structure.

In yet a further example, the support member may be provided with an aperture/window within which the circuitry 201 is disposed and the first flexible circuitry attached thereto may be bent, folded or passed through the aperture to the inner side of the support structure whilst the second flexible circuitry attached to the circuitry 201 may be bent, folded or passed through the aperture to the outer side of the support structure.

Figure 5 illustrates a yet further example of an apparatus 500 according to the first aspect of the present disclosure. The apparatus 500 comprises a support structure 501 having an aperture or window therethrough 510. The aperture is configured such that flexible circuitry can be disposed therein.

In this example, the first and second flexible circuitry sections 503 and 507 correspond to first and second major surfaces/sides of the same single flexible circuitry or substrate 511 which is disposed and mounted within the aperture 510. In effect, a double sided flexible circuitry 511 is provided with first flexible circuitry section 503 on one side disposed towards an inwards direction 504 and second flexible circuitry section 507 on the other side disposed towards an outward direction 508.

One or more first sensor(s) 505 (shown in outline) may be mounted/printed on a first under side of the double sided flexible circuitry 511 and disposed towards an inner side 504 of the support structure. One or more second sensor(s) 509 may be mounted on an opposing second upper side of the double sided flexible circuitry 511 and disposed towards an outer side 508 of the support structure.

The aperture, window or opening 510 could be provided through the support structure 501 such that the first sensor on the first side is exposed to the user's appendage. Also, the first sensor may be configured to monitor the user. Whereas the second sensor on the second side may be exposed to the external environment and the second sensor may be configured so as to monitor the external environment.

Although not shown, it is to be appreciated that one or more further first flexible circuitry sections (with one or more first sensor(s) thereon) may additionally be provided which extend beyond the aperture and are disposed on the inner side of the support structure. Similarly, one or more further second flexible circuitry sections (with one or more second sensor(s) thereon) could be provided disposed on the outer side of the support structure. In a yet further example, the double sided circuitry/substrate 511 may be rigid.

Although examples of the apparatus have been described above, and are further described below in terms of comprising various components, it should be understood that the components may be embodied as or otherwise controlled by a corresponding processing element or processor of the apparatus. In this regard, each of the components described above may be one of more of any device, means or circuitry embodied in hardware, software or a combination of hardware and software that is configured to perform the corresponding functions of the respective components.

Apparatuses in accordance with certain examples of the present disclosure may be configured for: portable use, wearable use (e.g. on a limb portion such as a: wrist, bicep, ankle ...) and wired or wireless communication (e.g. via a cellular network, WAN or short range wireless communication protocol). The apparatus may have additional functions beside communication and comprise:
user input interfaces (e.g. buttons, voice control, touch screen, as well as user input by user manipulation of the overall apparatus, e.g.
squeezing the apparatus between thumb and forefinger as discussed above) and
user output interfaces (e.g. audio-visual and haptic output devices).

In addition to providing sensor measurements and readings, examples of the apparatuses according to the present disclosure may additionally provide one or more audio/text/video communication functions (e.g. tele-communication, video-communication, and/or text transmission (Short Message Service (SMS)/ Multimedia Message Service (MMS)/emailing) functions), interactive/non-interactive viewing functions (e.g. web-browsing, navigation, TV/program viewing functions), music recording/playing functions (e.g. Moving Picture Experts Group-1 Audio Layer 3 (MP3) or other formats and/or (frequency modulation/amplitude modulation) radio broadcast recording/playing), downloading/sending of data functions, image capture function (e.g. using a (e.g. in-built) digital camera), and gaming functions. Electrical hardware and electrical components to effect such functionality may be provided in rigid circuitry 201 housed in the rigid section 304 of the apparatus.

Control of such functionality and other functionality of the apparatus may be provided by a controller.

Figure 6 schematically illustrates an example of a controller 600, i.e. to control the sensors and/or other functionality of apparatuses according to the present disclosure. The controller may be integrated in rigid circuitry 201 housed in the rigid section 304 of the apparatus.

Implementation of the controller 600 can be in hardware alone (e.g. processing circuitry 601 comprising one or more processors 602 and memory circuitry comprising one or more memory elements 603), have certain aspects in software including firmware alone or can be a combination of hardware and software (including firmware).

The controller 600 may be implemented using instructions 605 that enable hardware functionality, for example, by using executable computer program instructions 605 in a general-purpose or special-purpose processor that may be stored on a computer readable storage medium 603 (e.g. memory) or carried by a signal carrier, to be performed by such a processor.

In the illustrated example, the controller 600 is provided by a processor 602 and memory 603. Although a single processor and a single memory are illustrated in other implementations there may be multiple processors and/or there may be multiple memories some or all of which may be integrated/removable and/or may provide permanent/semi-permanent/ dynamic/cached storage.

The processor 602 is configured to read from and write to the memory 603. The processor 602 may also comprise an input interface 606 via which data, such as sensor signals and/or commands, are input to the processor 602 from at least one input device 608 (e.g. the at least first and second sensors) and an output interface 607 via which data and/or commands are output by the processor 602 to output device 609. The output device may comprise:
a transceiver via which data may be wirelessly communicated to other devices,
an audio output device such as a speaker
a visual output device such as a display, lights or other visual indication means
a haptic output device such as a vibrator.

The memory 603 stores a computer program 604 comprising computer program instructions 605 that control the operation of the apparatus when loaded into the processor 602. The computer program instructions provide the logic and routines that enable the apparatus to effect functionality, not least such as controlling the sensors, user input/output, wireless communication and also the method 700 discussed below with respect to Figure 7.

The computer program instructions 605 may arrive at the controller 600 via any suitable delivery mechanism. The delivery mechanism may be, for example, a non-transitory computer-readable storage medium 610, a computer program product, a memory device, a record medium such as a compact disc read-only memory or digital versatile disc, or an article of manufacture that tangibly embodies the computer program. The delivery mechanism may be a signal configured to reliably transfer the computer program.

Figure 7 illustrates a method 700 according to an example of the present disclosure.

In block 701, a manipulation of the apparatus 100 is detected. For example the outer sensor flex 107a could comprise a strain sensor printed thereon configured to detect bending and relative movement of the rigid section 304 and the flexible section 301a. Such an arrangement would be able to recognise user actuated manipulation of the support structure, e.g. a user squeezing the apparatus between his/her thumb and forefinger.

In block 702, operation of the apparatus is controlled in dependence on the detected manipulation. Such control may correspond to a user input or command to effect the operation of the apparatus.

The blocks may represent steps in a method and/or sections of instructions/code 605 in the computer program 604, i.e. such that the controller might be configured to cause the method 700 to be performed.

It will be understood that each block and combinations of blocks, can be implemented by various means, such as hardware, firmware, and/or software including one or more computer program instructions. For example, one or more of the procedures described above may be embodied by computer program instructions 605. In this regard, the computer program instructions which embody the procedures described above may be stored in the memory storage device 603 and performed by the processor 602.

As will be appreciated, any such computer program instructions may be loaded onto a computer or other programmable apparatus (i.e., hardware) to produce a machine, such that the instructions, when performed on the programmable apparatus, create means for implementing the functions specified in the blocks.

The computer program instructions may also be loaded onto a programmable apparatus to cause a series of operational steps to be performed on the programmable apparatus to produce a computer-implemented process such that the instructions which are performed on the programmable apparatus provide steps for implementing the functions specified in the blocks.

In various examples of the first aspect of the disclosure discussed above, the first section of flexible circuitry 103 a part of the support structure, i.e. it is coupled to and supported by an inner surface of the support structure 101 such that a single apparatus/device is provided. In various examples of a second aspect of the disclosure, the first section of flexible circuitry is instead separate of the support structure and separately attached to the appendage. For example the portion of flexible circuitry may be provided in a wearable sensor element that is a separate device to that of the support structure device. The wearable sensor element may be physically distinct/mechanically separated from the support structure such that the apparatus comprises two separate device/modules: the support structure and the wearable sensor element. The wearable sensor element may however be electrically coupled and in data communication with electronics and control circuitry housed in the support structure. For example one end of a flexible circuit may be physically coupled to the support structure to provide a data connection whereas the other end of the flexible circuit, comprising the first section of flexible circuitry, may be separate from support structure.

The provision of such two separate devices/modules in the apparatus of the present disclosure enables a light weight, thin wearable sensor element to be tightly attached to a user's appendage, thereby improving measurements made therefrom, whilst a heavier, thicker and more bulky support structure (comprising a power supply, user interface, electronics and circuitry for controlling the wearable sensor element) can be loosely attached. Advantageously, this improves the comfort of wearing the dual module apparatus (e.g. reducing: heat conduction, sweating under the more bulky support structure and obstructing upper skin blood circulation) whilst not adversely affecting the ability to detect accurate physiological measurements.

The wearable sensor element and the support electronics/control circuitry of the support structure may be configured to measure physiological measurements such as Heart rate (HR) & heart rate variability (HRV) for example using:
electroencephalographic (ECG) methods based on direct contact electrodes on the skin or capacitive contact,
opto-electrical photoplethysmography (PPG) measurements using a light source (LED) and photodetector (e.g. transistor/diode, PD) as receiver against the skin,
LED and Photo diode arrays as transmitter-receiver pairs against the skin, a camera as a detector,
ultrasonic: Laser Doppler Flowmetry (LDF)/Velocimetry (LDV),
radar on the wrist (Nanosecond Pulse Near Field Sensing, NPNS (300 MHz),
magnetic methods which utilise Giant-Magneto-Resistance (GMR) to measure Phonocardiographic (PCG) signals,
methods utilising an electrical coil (as a part of an oscillator),
methods utilising electromechanical film, e.g. Emfi(t)-foil, and
methods utilising an acoustic sensor based microphone.

Several artefacts make reliable HR/HRV measurement challenging, such as: movement, breathing and also possibly lighting environment in case of optical measurements. Various examples of the present disclosure seek to minimise the effect of such artefacts and improve the accuracy of HR/HRV measurements.

Figure 8 schematically illustrates a cross sectional view of an apparatus 800 according to an example of the second aspect of the present disclosure. The apparatus 800 comprises a support structure 801 and at least a first section of flexible circuitry 803. The first section of flexible circuitry 803 comprises circuitry mounted/printed/integrated thereon, such as sensors 805 and an interface 806 for communicating with an interface 807 of the support structure 801. One or more of the sensors 805 may be configured to monitor a user of the apparatus via direct physical contact with a user's appendage, e.g. wrist, so as to take a physiological measurement of the user. One or more of the sensors may be configured to monitor an external environment.

The first section of flexible circuitry is separate from the support structure, i.e. separately attached to the user's appendage (c.f. certain examples of the first aspect of the present disclosure wherein the first flexible circuitry section is attached to the support and thus attached to the appendage via the support structure's attachment mechanism, e.g. its band like structure.

The first section of flexible circuitry may be comprised in a wearable sensor element 810 which is separate from and distinct to the support structure 801. The sensor element is wearable in that it is attachable to the user's appendage and able to be carried and borne by the user on his/her appendage, such as a limb or wrist.

The wearable sensor element may comprise: electrodes, functional material that converts physical properties into an electrical signal, or other electronics e.g. transistors and passives.

The wearable sensor element may comprise: hard, flexible, stretchable structures and breathable materials, such as cloth or bandage material. The wearable sensor element may be tightly attached, light weight, thin and conformable to the user's appendage.

The provision of a separate wearable sensor element means that the apparatus is, in effect, a multi module apparatus comprising a first module, namely the support structure, and a separate module, namely the wearable sensor element. The two modules are separate in that they are not a part of the same single structure nor integrally formed together. Whilst the two modules may in certain examples be electrically coupled together (as mentioned above) they are nevertheless separated from one another in as much as they are mechanically separate/ mechanically de-coupled from one another so as to enable relative movement from one another. The provision of two such modules means that one of the modules can easily be replaced thereby readily providing the ability to customize device functions and style.

Moreover, each of the support structure 801 and the wearable sensor element 810 modules comprises its own attachment mechanism for respectively attaching the support structure and the wearable sensor element to the user's appendage. In example of Figure 8, a first attachment mechanism of the support structure comprises the band shaped support structure itself, which is dimensioned so as to enable it to be fitted around the user's appendage. Likewise, the second attachment mechanism of the wearable sensor element comprises the band shaped wearable sensor element itself dimensioned so as to be fitted around the user's appendage thereby securing the at least a portion of flexible circuitry against the appendage. In Figure 8 the support structure and the separate wearable sensor element 810 are each provided in the form of a bracelet, such that the apparatus is a dual bracelet device. Each bracelet may be adjustable to fit the user's appendage.

Any suitable attachment means may be employed to temporarily, semi-permanently and permanently attach the wearable sensor element/at least a portion of the first flexible circuitry to the user's appendage. Such means may include, for example: bands, bracelets, straps, bandages, stickers, tapes, plaster, fastening means, adhesive means, as well as tattoos. The means may be: adjustable, stretchable and conformable to a user's appendage to ensure an appropriate level of tightness of fit.

Advantageously, the provision of a separate support structure and wearable sensor element each having their own attachment mechanism enables the tightness of fit, or alternatively the looseness, of fit of each module to the user's appendage to differ. The ability to tightly fit the wearable sensor element allows the accuracy of measurements to be improved by tightly coupling electrodes and/or sensing elements to the user's appendage. For example a signal from optical heart rate measurement varies depending on the sensor's relative location to blood vessel, ambient light leakage to the photodetector and movement of the sensing element. Vertical displacement, or displacement perpendicular to the surface of the appendage by the wearable sensor element may be restricted by the attachment mechanism, e.g. an elastic force forcing the wearable sensor element in a direction towards the appendage. Whereas horizontal displacement, or displacement parallel to/tangential to the surface of the appendage by the wearable sensor element may by restricted by frictional forces. Thus the relative location of the wearable sensor element can be maintained and fixed.

The support structure 801 comprises a rigid portion 812 for supporting electronic components, such as interface 807, power supply 814, and electronics/circuitry 813 for controlling the wearable sensor element, i.e. its first section of flexible circuitry 803 and the sensors 805. The interface 807 may provide power as well as control signals to the wearable sensor element via the interface 806. Also, the interface 807 may receive sensor measurements from the wearable sensors element via the interface 806. The interfaces 806 and 807 may provide a wired connection (e.g. via cables or flexible circuitry such as FCB), a wireless connection (e.g. via radio frequency, NFC, IR or optical based wireless communication) or alternatively the interface may be via galvanic contacts such as sliding galvanic spring connectors for transferring data and energy to the wearable sensor element.

Since the support structure houses the majority of the hardware to control the wearable sensor element (e.g. a power supply, electronic components, user interface and circuitry for controlling the sensors of the wearable sensor element) the wearable sensor element can be made to be lighter than the support structure and/or and thinner than the support structure, i.e. having one or more dimensions less than those of the support structure. In certain examples, the support structure may have a mass 10 - 100 times greater than that of the wearable sensor element and a volume 10 - 100 times greater than that of the wearable sensor element. In certain other examples, the support structure may have a mass greater than 10 grams, e.g. ranging from 10 to 70 grams, with a volume of greater than 5 cm³, e.g. ranging from 5 cm³ to 300 cm³, whereas the wearable sensor element may have a mass of less than 2 grams, e.g. ranging from 0.1 - 2 grams, with a volume of less than 0.25 cm³, e.g. ranging from 0.01 to 0.25 cubic centimetres. Advantageously, this gives rise to less strain and pressure on the user's appendage when wearing the apparatus. The relatively low mass wearable sensor element can be kept in place with a relatively small force and thus can be tightly fitted to the user's appendage without undue force/digging into the user's appendage causing discomfort. Whereas the relatively higher mass support structure can be loosely fitted and allowed to move relative to the appendage, thus not obstructing upper skin blood circulation and giving higher levels of comfort.

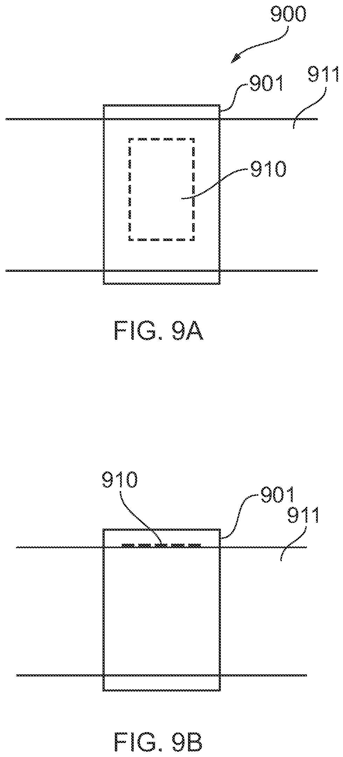

The wearable sensor element of Figure 8 is shown in the form of a band which encircles the user's appendage, likewise, at least the first section of flexible circuitry has a band like for factor encircling the user's appendage. It will be appreciated that other, e.g. non-band like, form factors of the flexible circuitry and wearable sensor element may be provided for example as shown in Figures 9A and 9B.

Figures 9A and 9B show a plan view and a side on view of an apparatus 900 comprising a support structure 901 having a band/bracelet type attachment mechanism which encircles a user's wrist 911. The apparatus also comprises a separate wearable sensor element 901, which is separate and distinct from the support structure. In this example, the wearable sensor element, which comprises at least a first section of flexible circuitry and one or more sensors as well as an interface to electronics and control circuitry of the support structure, takes the form of a patch/plaster having an adhesive based attachment mechanism for attaching the wearable sensor element to the wrist. The wearable sensor element is disposed between the underside of the support structure and the user's wrist. Such co-location of the support structure and the wearable sensor element facilitates their electrical/data communication with one another since shorter wires/electrical contacts or and shorter wireless range would be required.

The wearable sensor element could be configured to be tightly fitted to a user's wrist, thereby enabling its sensors to be tightly coupled and fixed in position against the user wrist to improve sensor measurement. Whereas the support structure could be more loosely fitted to the user's wrist and able to move relative to the wrist thereby improving user comfort in wearing the (relatively heavier and bulkier) support structure, particularly over prolonged periods of time.

The support structure could be configured to be loosely fitted to a user's wrist, thereby permitting its movement and loose skin contact avoiding sweating under the support structure making it possible for air to flow and excess humidity to escape underneath the support structure. Advantageously, this gives rise to less conducted heat from the support structure to the user's appendage. This allows for sensor measurements from body and environment to be more easily separated. E.g. the temperature changes of the main electronics unit and energy storage of the support structure have less effect on the body temperature measurements of the wearable sensor element. The energy storing unit in the relatively loosely fitted support part can be easily replaced, which provides a similar user experience to instant recharging.

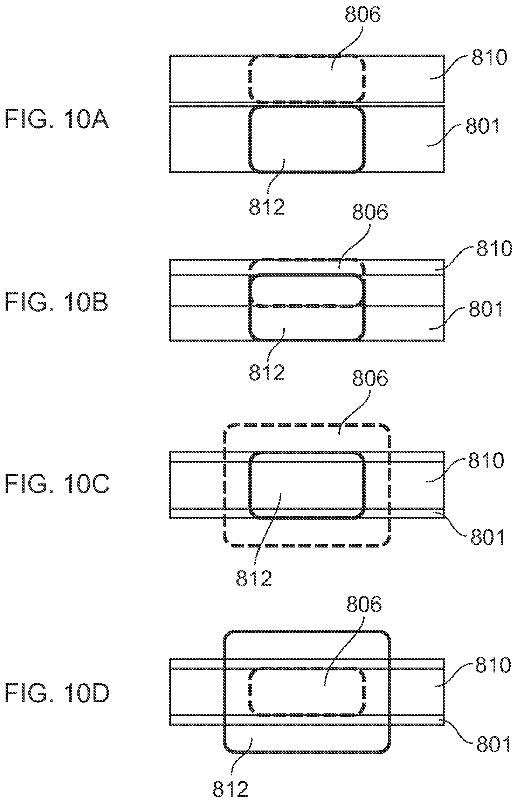

Figures 10A-D shows various configurations of possible relative locations (and sizes) of the wearable sensor element and the support structure when worn and in use.

In Figure 10A, the support structure 801 is disposed adjacent/side-by-side with the wearable sensor element 810 such that the wearable sensor element 810 does not cover the support structure 801 at all. In Figure 10B, the support structure 801 at least partially overlays the wearable sensor element 810. In Figure 10C, the support structure 801 substantially overlays/overlaps the wearable sensor element 810, with the exception of a portion of the wearable sensor element 806, which e.g. may comprise a sensor or interface, that extends beyond the overlaying support structure. In Figure 10D, the support structure 801 substantially entirely overlays the wearable sensor element 810. Moreover, a portion of the support structure 812 may be wider that the band like remainder of the support structure, such a portion may comprise electronics, circuitry, power supply and user interface for controlling the wearable sensor element.

In the above description, the wording 'connect', 'couple' and 'communication' and their derivatives mean operationally connected/coupled/in communication. It should be appreciated that any number or combination of intervening components can exist (including no intervening components).

Features described in the preceding description may be used in combinations other than the combinations explicitly described. For example, features described in the examples of the first aspect of the disclosure may be combined with the examples of the second aspect of the disclosure and vice versa. Although functions have been described with reference to certain features, those functions may be performable by other features whether described or not. Although features have been described with reference to certain examples, those features may also be present in other examples whether described or not.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one ..." or by using "consisting".

In this description, reference has been made to various examples. The description of features or functions in relation to an example indicates that those features or functions are present in that example. The use of the term 'example' or 'for example' or 'may' in the text denotes, whether explicitly stated or not, that such features or functions are present in at least the described example, whether described as an example or not, and that they can be, but are not necessarily, present in some or all other examples. Thus 'example', 'for example' or 'may' refers to a particular instance in a class of examples. A property of the instance can be a property of only that instance or a property of the class or a property of a sub-class of the class that includes some but not all of the instances in the class.

The above description describes some examples of apparatuses according to the present disclosure. However those of ordinary skill in the art will be aware of possible alternative structures and method features which offer equivalent functionality to the specific examples of such structures and features described herein above and which for the sake of brevity and clarity have been omitted from the above description. Nonetheless, the above description should be read as implicitly including reference to such alternative structures and method features which provide equivalent functionality unless such alternative structures or method features are explicitly excluded in the above description.

It should be appreciated that modifications to the examples presently described given can be made without departing from the scope of the invention as set out in the following claims.

## Claims

1. An apparatus (800) comprising:
a support structure (801) configured to at least partially enclose an appendage of a user, said support structure (801) defining an inner side and an outer side; and
a first section of flexible circuitry (803) disposed towards the inner side of the support structure (801);
wherein the first section of flexible circuitry (803) is separate from the support structure (801) and is comprised in a wearable sensor element (810) separate from the support structure (801);
wherein the support structure (801) comprises:
a first attachment mechanism for attaching the support structure to the appendage; and
wherein the wearable sensor element (810) comprises a second attachment mechanism, different from the first attachment mechanism, for attaching the wearable sensor element (810) to the appendage,
**characterised in that** the support structure (801) further comprise a rigid section (812) comprising electronic components (813) for controlling the wearable sensor element (810).

2. The apparatus of claim 1, wherein the first attachment mechanism of the support structure comprises one of:
a first bracelet, band or strap;
and wherein the second attachment mechanism of the wearable sensor element comprises one of:
a second bracelet, band or strap, or
an adhesive based attachment mechanism.

3. The apparatus of any preceding claim, wherein the first section of flexible circuitry at least one of:
comprises circuitry printed thereon;
comprises at least a first sensor;
comprises an array of sensors disposed along the length of the first section of flexible circuitry;
comprises at least a first sensor, disposed towards one side of the first section of flexible circuitry, configured to monitor the user;
comprises at least a second sensor, disposed towards another side of the first section of flexible circuitry, configured to monitor an external environment; and
extends around a substantial portion of the inner side of the support structure.

4. The apparatus of any preceding claim, wherein the support structure comprises a power supply.

5. The apparatus of any preceding claim, wherein the apparatus is configured such that:
the support structure substantially entirely overlays the wearable sensor element;
the support structure at least partially overlays the wearable sensor element; or
the support structure is disposed adjacent the wearable sensor element.

6. The apparatus of any preceding claim, wherein the apparatus comprises a second section of flexible circuitry disposed towards the outer side of the support structure.

7. The apparatus of any preceding claim, wherein the second section of flexible circuitry comprises at least a second sensor mounted thereon.

8. The apparatus of any preceding claim, wherein the second section of flexible circuitry at least one of:
comprises circuitry printed thereon;
comprises at least a second sensor;
comprises an array of sensors disposed along the length of the second section of flexible circuitry;
comprises at least a second sensor, disposed towards one side of the second section of flexible circuitry, configured to monitor an external environment; and
comprises at least a first sensor, disposed towards another side of the second section of flexible circuitry, configured to monitor the user; and
extends around a significant portion of the outer side of the support structure.

9. The apparatus of any of claims 6 - 8, wherein the first section of flexible circuitry is a portion of a first side of flexible circuitry and the second section of flexible circuitry is a portion of a second side of the flexible circuitry.

10. The apparatus of any of claims 6 - 9, wherein a substrate of the first section of flexible circuitry is different to a substrate of the second section of flexible circuitry.

11. The apparatus of any preceding claim, further comprising electronic hardware mounted in the rigid section.

12. The apparatus of any preceding claim, wherein the support structure comprises at least one of the following:
a flexible section,
a user manipulable section, and
an aperture through which at least a portion of the first section of flexible circuitry can pass.

13. The apparatus of any preceding claim, further comprising at least one sensor configured to detect user manipulation of a part of the apparatus.

14. The apparatus of any preceding claim, wherein a sensor signal from the at least one sensor is configured to control the apparatus.

15. A wearable device comprising the apparatus of any preceding claim.

## Patentansprüche

1. Vorrichtung (800), umfassend:
eine Trägerstruktur (801), die ausgestaltet ist, um eine Extremität eines Anwenders mindestens teilweise zu umschließen, wobei die Trägerstruktur (801) eine Innenseite und eine Außenseite definiert; und
einen ersten Anteil von flexibler Schaltung (803), der in Richtung der Innenseite der Trägerstruktur (801) angeordnet ist;
wobei der erste Anteil der flexiblen Schaltung (803) von der Trägerstruktur (801) getrennt vorliegt und in einem am Körper tragbaren Sensorelement (810) enthalten ist, das getrennt von der Trägerstruktur (801) vorliegt,
wobei die Trägerstruktur (801) umfasst:
einen ersten Befestigungsmechanismus zum Befestigen der Trägerstruktur an der Extremität; und
wobei das am Körper tragbare Sensorelement (810) einen zweiten Befestigungsmechanismus umfasst, der sich von dem ersten Befestigungsmechanismus unterscheidet, um das am Körper tragbare Sensorelement (810) an der Extremität zu befestigen,
**dadurch gekennzeichnet, dass** die Trägerstruktur (801) ferner einen starren Anteil (812) umfasst, der elektronische Komponenten (813) umfasst, um das am Körper tragbare Sensorelement (810) zu steuern.

2. Vorrichtung nach Anspruch 1, wobei der erste Befestigungsmechanismus der Trägerstruktur eines der folgenden umfasst:
ein erstes Armband, Band oder einen ersten Riemen;
und wobei der zweite Befestigungsmechanismus des am Körper tragbaren Sensorelements eines der folgenden umfasst:
ein zweites Armband, Band oder einen zweiten Riemen, oder
einen auf Klebstoff basierenden Befestigungsmechanismus.

3. Vorrichtung nach einem der vorhergehenden Ansprüche, wobei auf den ersten Anteil der flexiblen Schaltung mindestens eines der folgenden zutrifft:
umfasst darauf gedruckte Schaltung;
umfasst mindestens einen ersten Sensor;
umfasst eine Gruppierung von Sensoren, die entlang der Länge des ersten Abschnitts der flexiblen Schaltung angeordnet sind;
umfasst mindestens einen ersten Sensor, der in Richtung einer Seite des ersten Anteils der flexiblen Schaltung angeordnet ist und ausgestaltet ist, um den Anwender zu überwachen;
umfasst mindestens einen zweiten Sensor, der in Richtung einer anderen Seite des ersten Anteils der flexiblen Schaltung angeordnet ist und ausgestaltet ist, um eine äußere Umgebung zu überwachen; und
erstreckt sich um einen wesentlichen Abschnitt der Innenseite der Trägerstruktur.

4. Vorrichtung nach einem der vorhergehenden Ansprüche, wobei die Trägerstruktur eine Stromversorgung umfasst.

5. Vorrichtung nach einem der vorhergehenden Ansprüche, wobei die Vorrichtung so konfiguriert ist, dass:
die Trägerstruktur im Wesentlichen vollständig über dem am Körper tragbaren Sensorelement liegt;
die Trägerstruktur mindestens teilweise über dem am Körper tragbaren Sensorelement liegt; oder
die Trägerstruktur neben dem am Körper tragbaren Sensorelement angeordnet ist.

6. Vorrichtung nach einem der vorhergehenden Ansprüche, wobei die Vorrichtung einen zweiten Anteil von flexibler Schaltung umfasst, der in Richtung der Außenseite der Trägerstruktur angeordnet ist.

7. Vorrichtung nach einem der vorhergehenden Ansprüche, wobei der zweite Anteil der flexiblen Schaltung mindestens einen darauf montierten zweiten Sensor umfasst.

8. Vorrichtung nach einem der vorhergehenden Ansprüche, wobei auf den zweiten Anteil der flexiblen Schaltung mindestens eines der folgenden zutrifft:
umfasst darauf gedruckte Schaltung;
umfasst mindestens einen zweiten Sensor;
umfasst eine Gruppierung von Sensoren, die entlang der Länge des zweiten Abschnitts der flexiblen Schaltung angeordnet sind;
umfasst mindestens einen zweiten Sensor, der in Richtung einer Seite des zweiten Anteils der flexiblen Schaltung angeordnet ist und ausgestaltet ist, um eine äußere Umgebung zu überwachen; und
umfasst mindestens einen ersten Sensor, der in Richtung einer anderen Seite des zweiten Anteils der flexiblen Schaltung angeordnet ist und ausgestaltet ist, um den Anwender zu überwachen; und
erstreckt sich um einen wesentlichen Abschnitt der Außenseite der Trägerstruktur.

9. Vorrichtung nach einem der Ansprüche 6 bis 8, wobei der erste Anteil der flexiblen Schaltung ein Abschnitt einer ersten Seite der flexiblen Schaltung ist und der zweite Anteil der flexiblen Schaltung ein Abschnitt einer zweiten Seite der flexiblen Schaltung ist.

10. Vorrichtung nach einem der Ansprüche 6 bis 9, wobei ein Substrat des ersten Anteils der flexiblen Schaltung sich von einem Substrat des zweiten Anteils der flexiblen Schaltung unterscheidet.

11. Vorrichtung nach einem der vorhergehenden Ansprüche, ferner umfassend elektronische Hardware, die in dem starren Anteil montiert ist.

12. Vorrichtung nach einem der vorhergehenden Ansprüche, wobei die Trägerstruktur mindestens eines der folgenden umfasst:
einen flexiblen Anteil,
einen vom Anwender manipulierbaren Anteil und
eine Öffnung, die mindestens ein Abschnitt des ersten Anteils der flexiblen Schaltung passieren kann.

13. Vorrichtung nach einem der vorhergehenden Ansprüche, ferner umfassend mindestens einen Sensor, der ausgestaltet ist, um Anwendermanipulation eines Teils der Vorrichtung zu erkennen.

14. Vorrichtung nach einem der vorhergehenden Ansprüche, wobei ein Sensorsignal von dem mindestens einen Sensor ausgestaltet ist, um die Vorrichtung zu steuern.

15. Am Körper tragbares Gerät, welches die Vorrichtung gemäß einem der vorhergehenden Ansprüche umfasst.

## Revendications

1. Appareil (800) comprenant :
une structure de support (801) configurée pour entourer au moins partiellement un appendice d'un utilisateur, ladite structure de support (801) définissant un côté interne et un côté externe ; et
une première section de circuiterie souple (803) disposée vers le côté interne de la structure de support (801) ;
dans lequel la première section de circuiterie souple (803) est séparée de la structure de support (801) et est incluse dans un élément de capteur portable (810) séparé de la structure de support (801) ;
dans lequel la structure de support (801) comprend :
un premier mécanisme de fixation pour fixer la structure de support à l'appendice ; et
dans lequel l'élément de capteur portable (810) comprend un second mécanisme de fixation, différent du premier mécanisme de fixation, pour fixer l'élément de capteur portable (810) à l'appendice,
**caractérisé en ce que** la structure de support (801) comprend en outre une section rigide (812) comprenant des composants électroniques (813) pour commander l'élément de capteur portable (810).

2. Appareil selon la revendication 1, dans lequel le premier mécanisme de fixation de la structure de support comprend l'un des éléments suivants :
un premier bracelet, une première bande ou une première sangle ;
et dans lequel le second mécanisme de fixation de l'élément de capteur portable comprend l'un des éléments suivants :
un second bracelet, une seconde bande ou une seconde sangle, ou
un mécanisme de fixation par adhésif.

3. Appareil selon l'une quelconque des revendications précédentes, dans lequel la première section de circuiterie souple :
comprend une circuiterie imprimée sur cette dernière ; et/ou
comprend au moins un premier capteur ; et/ou comprend un réseau de capteurs disposés sur toute la longueur de la première section de circuiterie souple ; et/ou
comprend au moins un premier capteur, disposé vers un côté de la première section de circuiterie souple, configuré pour surveiller l'utilisateur ; et/ou
comprend au moins un second capteur, disposé vers un autre côté de la première section de circuiterie souple, configuré pour surveiller un environnement externe ; et/ou
s'étend autour d'une partie significative du côté interne de la structure de support.

4. Appareil selon l'une quelconque des revendications précédentes, dans lequel la structure de support comprend une alimentation électrique.

5. Appareil selon l'une quelconque des revendications précédentes, l'appareil étant configuré de telle sorte que :
la structure de support chevauche sensiblement entièrement l'élément de capteur portable ;
la structure de support chevauche au moins partiellement l'élément de capteurs portable ; ou
la structure de support soit disposée adjacente à l'élément de capteur portable.

6. Appareil selon l'une quelconque des revendications précédentes, l'appareil compren une seconde section de circuiterie souple disposée vers le côté externe de la structure de support.

7. Appareil selon l'une quelconque des revendications précédentes, dans lequel la seconde section de circuiterie souple comprend au moins un second capteur monté sur cette dernière.

8. Appareil selon l'une quelconque des revendications précédentes, dans lequel la seconde section de circuiterie souple :
comprend une circuiterie imprimée sur cette dernière ; et/ou
comprend au moins un second capteur ; et/ou comprend un réseau de capteurs disposés sur toute la longueur de la seconde section de circuiterie souple ; et/ou
comprend au moins un second capteur, disposé vers un côté de la seconde section de circuiterie souple, configuré pour surveiller un environnement externe ; et/ou
comprend au moins un premier capteur, disposé vers un autre côté de la seconde section de circuiterie souple, configuré pour surveiller l'utilisateur ; et/ou
s'étend autour d'une partie significative du côté externe de la structure de support.

9. Appareil selon l'une quelconque des revendications 6 à 8, dans lequel la première section de circuiterie souple est une partie d'un premier côté de circuiterie souple et la seconde section de circuiterie souple est une partie d'un second côté de la circuiterie souple.

10. Appareil selon l'une quelconque des revendications 6 à 9, dans lequel un substrat de la première section de circuiterie souple est différent d'un substrat de la seconde section de circuiterie souple.

11. Appareil selon l'une quelconque des revendications précédentes, comprenant en outre un matériel électronique monté dans la section rigide.

12. Appareil selon l'une quelconque des revendications précédentes, dans lequel la structure de support comprend au moins l'un des éléments suivants :
une section souple,
une section pouvant être manipulée par un utilisateur, et
un orifice à travers lequel au moins une partie de la première section de circuiterie souple peut passer.

13. Appareil selon l'une quelconque des revendications précédentes, comprenant en outre au moins un capteur configuré pour détecter une manipulation d'utilisateur d'une partie de l'appareil.

14. Appareil selon l'une quelconque des revendications précédentes, dans lequel un signal de capteur provenant de l'au moins un capteur est configuré pour commander l'appareil.

15. Dispositif portable comprenant l'appareil selon l'une quelconque des revendications précédentes.
